# EUROPÄISCHE PATENTSCHRIFT

(11) **EP 0 985 419 B1**
(45) Veröffentlichungstag und Bekanntmachung des Hinweises auf die Patenterteilung: **20.07.2005**
(21) Anmeldenummer: 99810775.9
(22) Anmeldetag: 27.08.1999
(51) Int. Cl.: A61M 5/168

(54) **Drucküberwachung eines bei einer Infusion oder Injektion dosiert zu verabreichenden Produktfluids**
Pressure monitoring of fluid delivered in doses during infusion or injection
Contrôle de pression d'un fluide, perfusé ou injecté, administré par dose

(30) Priorität: 08.09.1998 DE 19840992
(43) Veröffentlichungstag der Anmeldung: 15.03.2000
(73) Patentinhaber: Disetronic Licensing AG, 3401 Burgdorf (CH)
(72) Erfinder: Kipfer, Urs, 3432 Lützelflüh (CH); Imhof, Erich, 3427 Utzenstorf (CH)
(74) Vertreter: Schwabe - Sandmair - Marx

(56) Entgegenhaltungen:
- EP-A- 0 589 356
- WO-A-96/27398
- DE-C- 19 723 648
- US-A- 5 425 716

## Beschreibung

Die Erfindung betrifft die Überwachung des Drucks eines bei einer Infusion oder Injektion dosiert zu verabreichenden Produktfluids.

Bei einer Infusion oder Injektion eines Produktfluids kommt der exakten Dosierung der dabei verabreichten Produktdosis große Bedeutung zu, insbesondere bei der dosierten Verabreichung an Menschen. Erfolgt die Verabreichung weitestgehend automatisiert, wie beispielsweise in der Insulinbehandlung mit tragbaren Infusionspumpen, so ist die Überwachung der ordnungsgemäßen Produktverabreichung wünschenswert, wenn nicht sogar unerlässlich. Eine wesentliche Möglichkeit der Überwachung ist die Überwachung des Drucks des Produktfluids, da bei ordnungsgemäßer Funktion der mechanischen und elektronischen Komponenten eines Infusions- oder Injektionsgeräts auf eine ordnungsgemäße Produktverabreichung geschlossen werden kann, solange der Fluiddruck eine vorgegebene Druckspanne einhält. Bei Nichteinhaltung kann auf eine Okklusion oder eine Leckage im Fluidführungssystem geschlossen werden. Leckagen und Okklusionen stellen insbesondere bei der automatisierten Verabreichung von Wirkstofflösungen im medizinischen oder tiermedizinischen Bereich wegen der Gefahr des Nichterkennens oder des zu späten Erkennens ein Risiko dar.

Infusionspumpen, bei denen der Druck des zu verabreichenden Produktfluids überwacht wird, um Verstopfungen im Fluidführungssystem der Pumpen zu erkennen, sind beispielsweise aus der US-PS 4,562,751 und der WO 96/27398 bekannt. Diese beiden Druckschriften betreffen Infusionspumpen, bei denen ein Produktfluid mittels eines Kolbens aus einem das Produkt enthaltenden Behältnis durch Verdrängung gefördert wird. Der Kolben ist im Behältnis geradverschiebbar und wird über einen parallel zu einer Verschiebeachse des Kolbens angeordneten Spindeltrieb angetrieben. Dabei ist ein Spindelantriebsglied in einem Gehäuse der Infusionspumpe um seine Längsachse drehbar und in Richtung seiner Längsachse verschiebbar gelagert. Eine als Abtriebsglied wirkende, auf der Spindel laufende Mutter ist im Gehäuse verdrehgesichert und mit dem Kolben gekoppelt, derart, dass sie den Kolben bei ihrer eigenen Geradverschiebung mitnimmt.

Mit ihrem freien Ende an einer Stirnseite stößt die Spindel nach der US-PS 4,562,751 gegen einen Druckschalter, der bei Überschreiten eines Schwelldrucks die Pumpe stillsetzt. Dieser Schwelldruck wird dann erreicht, wenn der Kolben im Behältnis nicht oder nur mit übermäßig großer Kraft verschoben werden kann. In solch einem Fall bewegt sich die Spindel aufgrund ihrer Drehbewegung in der Mutter entgegen der Verschubrichtung des Kolbens gegen den Druckschalter.

Als Nachteil dieser Art der Drucküberwachung wird in der WO 96/27398 angeführt, dass bei Verwendung solch eines Hochdruck-Begrenzungsschalters eine Okklusion nicht rechtzeitig angezeigt wird, da zunächst einmal mehrere Produktdosen, die hintereinander verabreicht werden sollten, sozusagen auflaufen müssen, um den Schwelldruck für den Schalter aufzubauen. Statt der Verwendung eines Hochdruck-Begrenzungsschalters wird durch die WO 96/27398 die Verwendung eines Kraftsensors vorgeschlagen, der einen Messwert ausgibt, der der gemessenen Kraft und damit dem Fluiddruck im Behältnis proportional ist. Die Drucküberwachung besteht darin, dass der Fluiddruck durch Messung der Reaktionskraft, die der Kolben bei seinem Vorschieben im Behältnis ausübt, zu zwei unterschiedlichen Zeitpunkten vom Kraftsensor aufgenommen wird und die beiden vom Kraftsensor ausgegebenen Messwerte miteinander verglichen werden. Dabei findet eine erste Messung bei der Verabreichung des Produktfluids und eine zweite Messung später vor Verabreichung der nächsten Produktdosis statt. Bei Unterschreiten einer vorgegebenen Differenz der beiden Messwerte werden eine Okklusion angezeigt und ein Alarmsignal aktiviert. Ist die Differenz größer als der vorgegebene Differenzwert, so zeigt dies an, dass der Druck im Behältnis gefallen ist und Produktfluid tatsächlich und in der erforderlichen Weise verabreicht wird. Diese Art der Drucküberwachung nimmt reichlich Zeit in Anspruch. Ferner sind lediglich Okklusionen feststellbar.

Aus der EP-A-0 589 356 ist eine Vorrichtung zur Überwachung des Drucks eines Druckfluids bekannt. Zum Zwecke einer Infusion aus einem Behältnis durch Vorschieben eines in dem Behältnis aufgenommenen Kolbens wird das Produktfluid dosiert ausgegeben. Ein Sollwert wird vorgegeben und mit einem Ist-Wert verglichen. Die Vorrichtung enthält ein Behältnis, das in das eine Gehäuse der Vorrichtung aufgenommen wird, wobei das Behältnis das Produktfluid enthält. Ein Kraftsensor misst eine als Maß für den Druck dienende, vom Kolben auf das Gehäuse ausgeübte Reaktionskraft. Dieser Kraftsensor gibt einen Messwert bezüglich des gemessenen Drucks aus. Eine Steuerung vergleicht den Messwert mit einem vorgegebenen Vergleichswert. Ein Sollwert wird in einem Speicher der Steuerung verfügbar gehalten und für einen Soll-/Ist-Vergleich mit der gemessenen Reaktionskraft, d. h. dem Messwert, verwendet, um aufgrund dieses Vergleiches die Reaktionskraft nachzuregeln.

Die Erfindung hat es sich zur Aufgabe gemacht, eine Drucküberwachung eines bei einer Infusion oder Injektion dosiert zu verabreichenden Produktfluids zu ermöglichen, bei der Fehlfunktionen möglichst rasch und zuverlässig festgestellt werden.

Die Erfindung geht von einer Überwachung des Drucks eines bei einer Infusion oder Injektion dosiert zu verabreichenden Produktftuids aus, bei der das Produktfluid aus einem Behältnis durch Vorschieben eines in dem Behältnis aufgenommenen Kolbens ausschüttbar ist bzw. ausgeschüttet wird. Als Produktfluid wird insbesondere eine flüssige Wirkstofflösung verstanden. Das Behältnis ist in einem Gehäuse aufgenommen, oder es wird durch das Gehäuse selbst gebildet. Als Maß für den Fluiddruck wird eine vom Kolben beim Vorschieben auf das Gehäuse ausgeübte Reaktionskraft gemessen und einer Steuerung für einen Antrieb des Kolbens zugeführt. Die Steuerung vergleicht diese gemessene Reaktionskraft mit einer vorgegebenen Vergleichskraft und steuert den Antrieb des Kolbens unter Berücksichtigung des daraus resultierenden Vergleichsergebnisses.

Die Vergleichskraft ist ein Sollwert für die Reaktionskraft, und es wird ein unmittelbarer Soll/Ist-Vergleich zwischen der gemessenen Reaktionskraft und ihrem Sollwert durchgeführt. Dieser wenigstens eine Sollwert ist gespeichert und zum Zwecke des Soll/Ist-Vergleichs mit der im Zuge einer Ausschüttung, insbesondere einer Verabreichung oder einem Primen, aktuell gemessenen Reaktionskraft für die Steuerung ständig abrufbar.

Indem von der Steuerung der Vergleich unmittelbar zwischen der aktuell gemessenen Reaktionskraft und deren Sollwert angestellt wird, d.h. die Differenz aus Soll und Ist überwacht wird, kann die Reaktionszeit im Falle einer bei der Überwachung festgestellten Fehlfunktion so kurz wie nur möglich gehalten werden.

Vorzugsweise begnügt sich die Steuerung mit der Überwachung bzw. Kontrolle der tatsächlichen Reaktionskraft und reagiert nur im Falle einer Überschreitung einer vorgegebenen, maximal zulässigen Differenz von Soll und Ist mit dem Stillsetzen des Antriebs und zweckmäßigerweise auch mit der Auslösung eines Alarms. Von dieser erfindungsgemäßen Berücksichtigung im Rahmen der Antriebssteuerung abgesehen kann die Steuerung ein Programm abfahren, wie dies übliche Steuerungen auch tun.

In einer besonders bevorzugten Ausführungsform ist auf dem Weg des Produktfluids vom Auslass des Behältnisses bis zur Austrittsstelle, beispielsweise dem vorderen Ende einer Infusionsnadel, ein Ventil angeordnet, das einen definierten Druckabfall bewirkt. Der Druckabfall wird vorzugsweise so gewählt, dass ein Auslaufen von Produktfluid aus dem Behältnis aufgrund der Schwerkraft bei allen im praktischen Gebrauch der Vorrichtung auftretenden Verhältnissen sicher verhindert wird. Bezüglich der Bauweise und Anordnung solch eines Ventils wird auf die deutsche Patentanmeldung Nr. 197 23 648 verwiesen, deren Lehre hiermit in Bezug genommen wird. Das Ventil wird vorzugsweise so nahe wie möglich bei der Austrittsstelle des Produktfluids angeordnet, um das gesamte Fluidleitungssystem in eine Lecküberwachung mit einzuschließen. Falls das Ventil, was einer ebenfalls bevorzugten Ausführungsform entspricht, im Strömungsweg des Produktfluids unmittelbar hinter dem Behältnisauslass angeordnet ist, so kann immerhin noch eine Leckage zwischen dem Kolben und dem Ventil festgestellt werden.

Zusätzlich noch wirkende Kräfte sind gegenüber der Ventilwirkung vernachlässigbar. Als Sollwert wird diejenige Reaktionskraft verwendet, die sich aufgrund des Nennwerts für den Druckabfall von Ventilen einer Ventilbaureihe ergibt. Auf diesen Sollwert ist die Steuerung herstellerseitig eingestellt. Der zulässige Bereich für die tatsächliche Reaktionskraft wird derart vorgegeben, dass er dem Bereich der Reaktionskraft entspricht, der sich aufgrund der Streuung des Nennwerts für den Druckabfall der Ventile der verwendeten Baureihe ergibt. Dieser Streubereich um den Sollwert wird nach oben und unten noch erweitert, um auch Streuungen bei anderen Komponenten, beispielsweise den verwendeten Antrieben, zu erfassen. Allerdings ist auch hier die Ventilstreuung in erster Linie das Maß. Zur Bildung der entsprechenden Schwellwerte in Bezug auf eine Okklusion und eine Leckage können die Streubereiche um Toleranzmargen erweitert sein. Eine Okklusion wird dann angenommen, wenn die gemessene Reaktionskraft aus dem derart vorgegebenen Sollwertbereich nach oben herausfällt. Eine Leckage wird angenommen, wenn sie aus dem derart vorgegebenen Sollwertbereich nach unten herausfällt. Insbesondere zur Leckageüberwachung ist das Ventil mit definiertem Druckabfall von Vorteil.

Ist solch ein Ventil nicht vorhanden oder ist der durch das Ventil erzeugte Druckabfall nur so groß, dass die darüberhinaus noch wirkenden Kräfte nicht in guter Näherung vernachlässigt werden dürfen, so können auch mehrere für eine Ausschüttung zu beachtende Sollwerte mit dazugehörigen Ober- oder Untergrenzen vorgegeben werden. Ein erster Sollwert kann dann für diejenige Kraft vorgegeben sein, die aufgebracht werden muss, um den Kolben aus einer Ruheposition heraus im Behältnis vorzuschieben. Diese Kraft entspricht im wesentlichen derjenigen, die zur Überwindung der Haftreibung zwischen dem Kolben und der Behältniswandung und von Strömungswiderständen aufgebracht werden muss. Da die Vorschubgeschwindigkeit zum Ausschütten des Produktfluids im allgemeinen konstant ist, übt der Kolben in der stationären Phase seiner Vorschubbewegung bei gefülltem Katheter auch eine konstante Reaktionskraft auf sein Stützlager, das Gehäuse, aus. Ein zweiter Sollwert entspricht dann im wesentlichen der Kraft, die zur Überwindung der Kolbengleitreibung und der Strömungswiderstände, die das Produktfluid auf seinem Weg vom Behältnis bis zu einer Austrittsstelle erfährt, erforderlich ist. Solche Strömungswiderstände stellen beispielsweise ein verengter Auslass am Behältnis, der sich daran anschließende Katheter und eine an einem freien Ende des Katheters angeschlossene Infusionsnadel dar. Eine Okklusion wird vorzugsweise dann angenommen, wenn die gemessene Reaktionskraft um einen vorgegebenen, maximalen Betrag größer als der erste Sollwert ist. Eine Leckage wird vorzugsweise dann angenommen, wenn die gemessene Reaktionskraft beim Vorschieben des Kolbens um einen vorgegebenen Betrag geringer als der zweite Sollwert ist.

Es können auch Sollwerte in Form eines Sollwertverlaufs über der Zeit bzw. dem Verschiebeweg vorgegeben sein, den der Kolben bei einer Verabreichung aus seiner Position unmittelbar vor der Verabreichung bis in seine Position unmittelbar am Ende der Verabreichung durchfährt. Der wenigstens eine Sollwert ist in diesem Fall ein Wert eines kompletten Sollwertprofils bzw. -verlaufs. Andere Sollwerte des Profils können ebenfalls zu Vergleichszwecken herangezogen werden.

Ein Primen des Kolbens bzw. der Vorrichtung kann manuell beendet oder aber vollautomatisch durchgeführt werden. Beim Primen wird der Kolben aus einer anfänglichen Einbauposition im Behältnis so weit vorgeschoben, bis Produktfluid an der Austrittsstelle austritt. Nach dem Primen sind somit alle fluidführenden Teile, im wesentlichen das Behältnis, der angeschlossene Katheter und die Infusionsnadel, mit Produktfluid gefüllt, und es kann dann die erste dosierte Verabreichung erfolgen. Der Abschluss des Primens kann im Zuge der erfindungsgemäßen Drucküberwachung festgestellt werden, indem die gemessene Reaktionskraft darauf überwacht wird, ob sie nach dem Austritt von Produktfluid mit dem vorgenannten zweiten Sollwert übereinstimmt oder nicht. Bei Anordnung eines Ventils zwischen dem Auslass des Behältnisses und der Nadel entspricht dieser Sollwert in guter Näherung ebenfalls der durch den Druckabfall zwischen Ventileinlass und Ventilauslass erzeugten Reaktionskraft.

Durch Vergleich der gemessenen Reaktionskraft mit dem wenigstens einen Sollwert kann auch der Füllzustand des Behältnisses automatisch ermittelt werden. Die Soll-Position des Kolbens entlang der Verschiebeachse des Kolbens steht der Steuerung von Hause aus zur Verfügung, da sie den Kolbenantrieb, vorzugsweise ein Linearantrieb, positionsgenau bezogen auf eine Plattform steuert. Ein erstes Anstoßen des Kolbenantriebs an den Kolben kann durch Vergleich der gemessenen Reaktionskraft mit dem wenigstens einen Sollwert festgestellt werden, insbesondere bei Verwendung des Ventils.

In ebenfalls bevorzugter Ausführung wird der Kolbenantrieb von der Steuerung so voreingestellt, dass er beim ersten Anfahren gegen den Kolben sich selbst stillsetzt. In diesem Fall kann die Kolbenposition und damit der Füllzustand in besonders einfacher Weise aus der beim Stillsetzen bekannten Position des Antriebsmotors ermittelt werden. Die Verwendung eines sich selbst stillsetzenden Motors, insbesondere ein Schrittmotor mit vorgegebener oder voreingestellter oder einstellbarer Start-Stop Frequenz, ist grundsätzlich zur Füllstandsermittlung möglich und generell vorteilhaft, d.h. auch ohne die im Zusammenhang mit dem Kraftvergleich beschriebenen weiteren Merkmale der Erfindung. Die Start-Stop Frequenz ist die maximale Frequenz, im Falle eines Schrittmotors die maximale Pulsfrequenz, mit welcher der Motor bei gegebener Ansteuerung anläuft. Sie ist bei gegebenem Motor von dem von dem Motor aufzubringenden Moment abhängig und wird daher im Allgemeinen als momentenabhängige Frequenkennlinie dargestellt.

Eine Drucküberwachung beim Primen wird vorteilhafterweise zur Gewinnung wenigstens eines Sollwerts für die Reaktionskraft genutzt, der für jede Vorrichtung individuell ist. Beim Primen selbst werden die dabei gemessenen Reaktionskräfte mit dem wenigstens einen Sollwert verglichen, der bereits vom Hersteller vorgegebenen und vorzugsweise in einem nicht löschbaren Permanentspeicher für die Steuerung abrufbar abgelegt ist.

Eine beim Primen gemessene Reaktionskraft wird als individueller Sollwert anstatt des herstellerseitig vorgegebenen Sollwerts für die Drucküberwachung bei der Verabreichung verwendet, falls die beim Primen gemessene Reaktionskraft innerhalb des zulässigen Sollwertbereichs liegt. In dieser Ausführungsform ist die Steuerung adaptiv. Durch die adaptive Steuerung wird die Sensibilität der Drucküberwachung erhöht.

Besonders bevorzugt wird die erfindungsgemäße Drucküberwachung bei tragbaren Infusionspumpengeräten verwendet, wie sie insbesondere in der Insulinbehandlung eingesetzt werden. Bei dieser Art von Infusionsgeräten erfolgt die Verabreichung des Produkdluids, beispielsweise Insulin, nahezu kontinuierlich, indem in kurzen zeitlichen Abständen über einen längeren Zeitraum geringste, genau dosierte Produktdosen im Zuge vieler Einzelausschuttungen verabreicht werden. Vollkommen kontinuierlich ablaufende Infusionen sollen jedoch wie Einzelinjektionen auch stets vom Begriff der Verabreichung mit umfasst sein.

Ein bevorzugtes Ausführungsbeispiel der Erfindung wird nachfolgend anhand von Figuren erläutert. Es zeigen:
- Fig. 1: einen Längsschnitt einer Infusionspumpe mit Drucküberwachung,
- Fig. 2: einen Kraftsensor für die Pumpe nach Figur 1,
- Fig. 3: eine Verschiebeplattform der Pumpe nach Figur 1 mit dem Kraftsensor und
- Fig. 4: einen Standard-Sollwertverlauf und einen individuellen Sollwertverlauf, wie er bei einem Primen ermittelt wird.

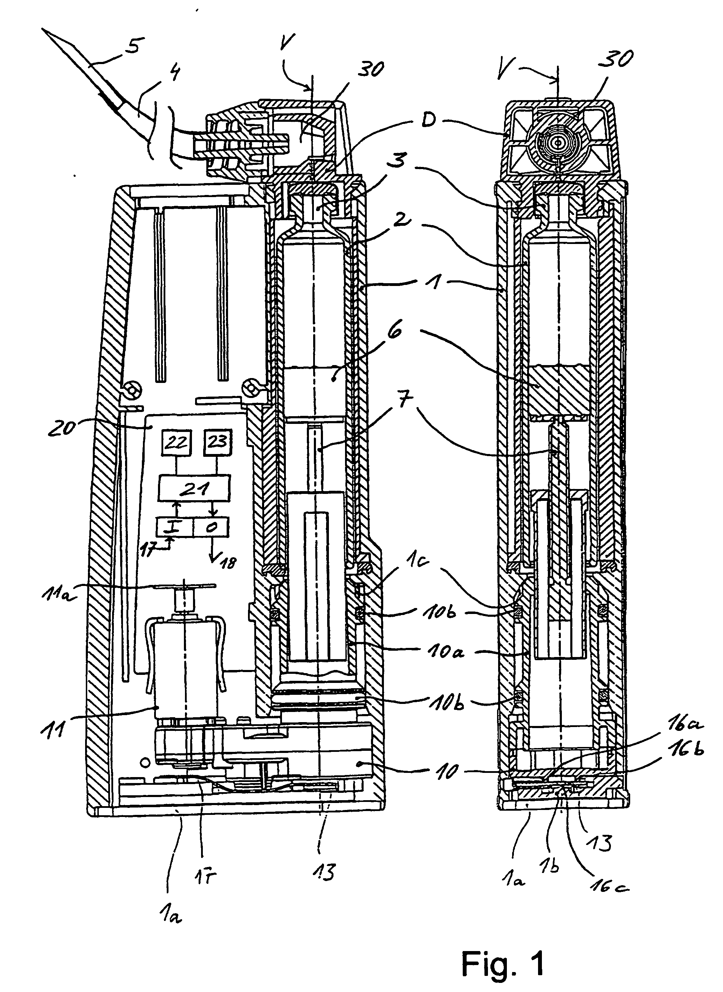

Fig. 1 zeigt eine tragbare Infusionspumpe für die Insulinbehandlung. Die Pumpe, insbesondere deren Antrieb, wird in der deutschen Patentanmeldung Nr. 197 17 107 beschrieben. Die Lehre dieser Anmeldung wird ergänzend in Bezug genommen.

In einem Pumpengehäuse 1 ist ein Behältnis in Form einer Ampulle 2 aufgenommen. Die Ampulle 2 ist mit Insulin gefüllt. Ein Kolben 6 ist in der Ampulle 2 in eine Vorschubrichtung auf einen Ampullenauslass 3 zu geradverschiebbar aufgenommen. Der Vorschub des Kolbens 6 wird durch Andruck eines als Gewindestange ausgebildeten Abtriebsglieds 7 auf eine rückwärtige Fläche des Kolbens 6 bewirkt. Das Abtriebsglied 7 ist Teil eines Spindeltriebs, der teleskopierbar zweistufig ausgebildet ist. Die Erfindung ist bezüglich des Kolbenantriebs jedoch hierauf nicht beschränkt.

Beim Vorschieben des Kolbens 6 entlang einer Verschiebeachse V wird Insulin über einen am Auslass 3 angeschlossenen Katheter 4 und eine am vorderen freien Ende des Katheters 4 befestigte Infusionsnadel 5 ausgeschüttet. Um in der Ampulle 2 einen definierten Basisdruck einzustellen, ist auf dem Strömungsweg des Insulins ein Ventil 30 angeordnet. Solch ein Ventil kann nicht nur zur Definition eines im Fluidführungssystem 2 - 5 stets herrschenden Basisdrucks verwendet werden, sondern auch zum Verhindern einer Selbstentleerung der Ampulle 2 unter dem Eigengewicht der Flüssigkeitssäule im Fluidführungssystem 2 - 5. Bevorzugterweise ist das Ventil 30 so ausgelegt, dass es solch eine unerwünschte Selbstentleerung unter den im praktischen Gebrauch der Infusionspumpe auftretenden Bedingungen sicher verhindert. Im Ausführungsbeispiel ist das Ventil 30 in einem abnehmbaren Gehäusedeckel D aufgenommen, mit dem ein Ampullenschacht nach Einsetzen der Ampulle 2 verschlossen wird.

Der Antrieb des Abtriebsglieds 7 erfolgt mittels eines Drehmotors 11 über ein Untersetzungsgetriebe auf den Spindeltrieb mit dem Abtriebsglied. In Bezug auf den Spindeltrieb und das Untersetzungsgetriebe wird auf die deutsche Patentanmeldung Nr. 197 17 107 verwiesen. Zumindest die Gewindestange 7 ist im Gehäuse 1 verdrehgesichert geradgeführt, so dass ein Drehantreiben von zwei vorgelagerten Antriebsgliedern des Spindeltriebs die das Abtriebsglied 7 hülsenförmig umgeben, einen Vorschub der Gewindestange 7 bewirkt.

Der Spindeltrieb ist zusammen mit dem Getriebe und dem Motor 11 auf einer Verschiebeplattform 10 gelagert, die ihrerseits als Ganzes im Gehäuse 1 verdrehgesichert geradverschiebbar in und gegen die Vorschubrichtung des Kolbens gelagert ist. Es wäre grundsätzlich auch möglich, den Motor 11 gehäusefest anzuordnen, ebenso wäre eine gehäusefeste Anordnung des Motors 11 zusammen mit dem Getriebe möglich. In diesem Fall müsste zwischen der gehäusefesten Antriebskomponente und der dann auf der Verschiebeplattform 10 gelagerten Eingangsstufe ein entsprechender Verschiebeeingriff, beispielsweise eine sich längs der Vorschubrichtung über eine entsprechende Länge erstreckende Stirnradverzahnung, vorgesehen sein.

Um etwaige Fehlfunktionen im Fluidführungssystem 2, 3, 4, 5 feststellen zu können, wird der Druck des Produktfluids, insbesondere der Fluiddruck in der Ampulle 2, überwacht. Für die Drucküberwachung wird eine vom Kolben 6 auf das Gehäuse 1 ausgeübte Reaktionskraft mittels eines Kraftsensors 13 gemessen und mit einem vorgegebenen Sollwert für die Reaktionskraft verglichen.

Als Reaktionskraft wird mittels eines Kraftsensors 13 die Kraft gemessen, die vom Kolben 6 auf die Gewindestange 7 und über den Spindeltrieb auf die Verschiebeplattform 10 und infolge deren Verschiebbarkeit auf den Kraftsensor 13 ausgeübt wird. Zu diesem Zweck ist die Verschiebeplattform 10 an Gehäusewandungen im Gehäuse 1 schwimmend gelagert.

Hierzu ist die Verschiebeplattform 10 mit einer Plattformhülse 10a in einem diese Hülse 10a umgebenden Gehäusehülsenteil längsverschiebbar mittels elastischen Lagerelementen 10b in Form eines Paars von O-Ringen, beispielsweise Gummiringe, abgestützt. Die Plattformhülse 10a umgibt als Antriebshülsen ausgebildete Antriebsglieder des Spindeltriebs. Ein Kontakt zwischen der Verschiebeplattform 10 und dem Gehäuse 1 wird nur über diese O-Ringe 10b hergestellt. Eine Verschiebebewegung entlang der Verschiebeachse V findet zwischen der Verschiebeplattform 10 und dem Gehäuse 1 nur im Rahmen der elastischen Verformung der O-Ringe 10b statt. Die O-Ringe 10b selbst werden weder gegenüber dem Gehäuse 1 noch gegenüber der Verschiebeplattform 10 verschoben, sondern lediglich elastisch verformt. Durch diese Ausbildung der schwimmenden Lagerung werden die Reibungskräfte minimiert und die bei dem Verschieben des Kolbens 6 aufgebrachte Kraft weitestgehend unverfälscht auf den Kraftsensor 13 übertragen. Die O-Ringe 10b sind in umlaufenden Nuten der Plattformhülse 10a aufgenommen und auf diese Weise gegenüber diesen beiden sich gegenüberliegenden Flächen des Gehäuses 1 und der Plattformhülse 10a durch Formschluss einerseits und Kraftschluss andererseits fixiert. Sie könnten jedoch auch mit einer dieser beiden Flächen stoffschlüssig verbunden sein, und sie könnten auch bei entsprechender Montage zwischen den beiden gegeneinander sich bewegenden Flächen zusammengedrückt und derart nur kraftschlüssig gehalten werden. Allerdings sollte dennoch gewährleistet sein, dass mit Ausnahme der elastischen Verformungskräfte keine weiteren Reibungskräfte beim Verschieben der Verschiebeplattform 10 wirken.

Der Kraftsensor 13 ist zwischen einer rückwärtigen Stirnfläche der Verschiebeplattform 10 und einer dieser rückwärtigen Stirnfläche gegenüberliegenden Wandung des Gehäuses 1 angeordnet. Er ist ferner in der Flucht der Verschiebeachse V des Kolbens 6 angeordnet, so dass die längs der Verschiebeachse V des Kolbens 6 wirkende Reaktionskraft unmittelbar auf den Kraftsensor 13 wirkt. Die Reaktionskraft wird symmetrisch bezüglich der Verschiebeachse V eingeleitet. Ein Kippmoment durch die Reaktionskraft kann daher nicht entstehen.

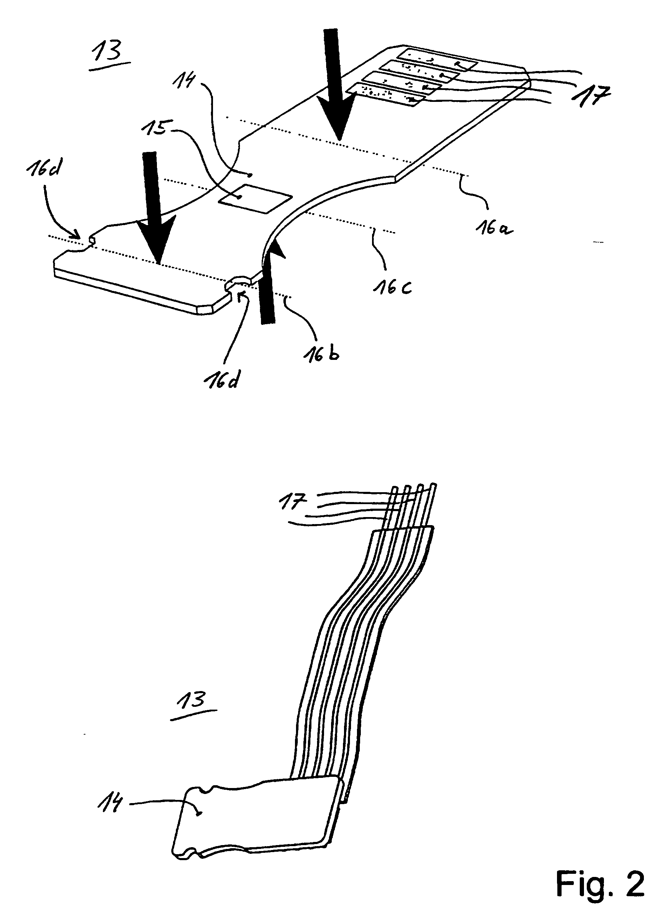

In Figur 2 ist der Kraftsensor 13 allein dargestellt. Er wird durch einen Biegebalken 14 gebildet, auf dem wenigstens an einer Balkenoberfläche ein Dünnfilm-Dehnungsmessstreifen 15 aufgebracht ist. Eine Messwertverstärkung könnte durch Aufbringen auf beiden sich gegenüberliegenden Balkenoberflächen erzielt werden. Vier Leitungen einer Brückenschaltung sind mit 17 bezeichnet. In Ausbildung des Biegebalkens sind an der einen Balkenoberfläche voneinander parallel beabstandet zwei Stege 16a und 16b (Figur 1) als plattformseitige, linienförmige Auflagen angedeutet, zwischen denen der Balken 14 mit dem Dehnungsmessstreifen 15 durch die vom Kolben 6 ausgeübte Reaktionskraft gebogen wird. Zur exakten Definition des Ortes der Einleitung der Reaktionskraft ragt von einer Bodenplatte 1b von der den beiden Stegen 16a und 16b gegenüberliegenden Unterseite des Gehäuses 1 exakt in der Mitte zwischen diesen beiden Stegen 16a und 16b ein weiterer Steg 16c ab (Figur 1), dessen linienförmige Auflage in Fig. 2 angedeutet ist. Die linienförmige Auflage des dritten Stegs 16c liegt in der Flucht der Verschiebeachse V und parallel zu den Stegen 16a und 16b. Die drei Stege 16a, 16b und 16c sind im Auflagebereich im Querschnitt rund, so dass die Reaktionskraft entlang der Stege 16a und 16b möglichst exakt linienförmig eingeleitet wird und auch die Auflagerlast linienförmig bei dem Steg 16c auf den Biegebalken 14 wirkt. Andere Querschnittsformen, die dies bewirken oder annähern, sind auch geeignet. Andere Sensoren, beispielsweise piezo-resistive Sensoren, wären statt Dehnungsmessstreifen im Rahmen eines statischen Messverfahrens ebenfalls verwendbar.

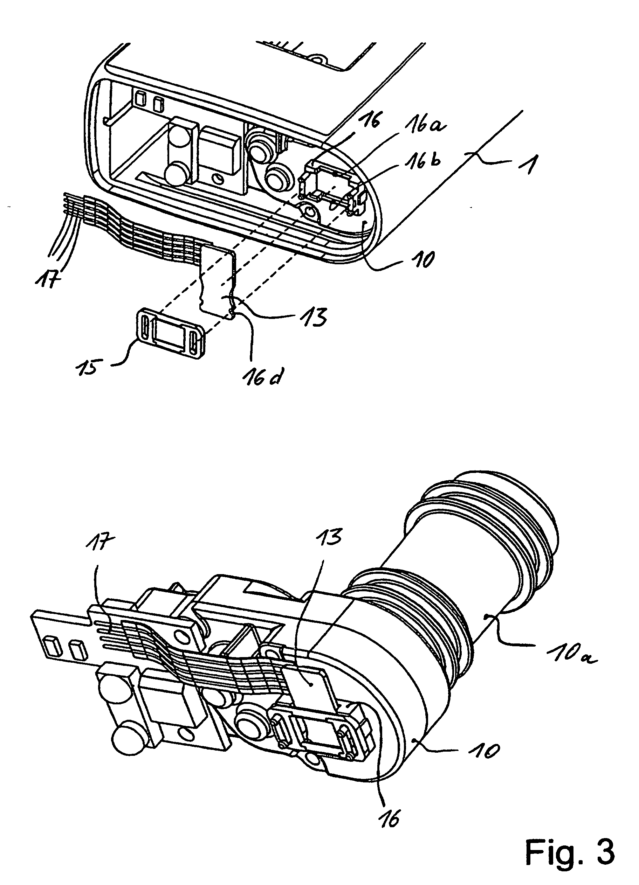

Die Anordnung des Kraftsensors 13 an der Verschiebeplattform 10 ist am besten in der Zusammenschau der beiden Zeichnungen der Figur 3 zu erkennen. Dabei zeigt die obere Zeichnung die Verschiebeplattform 10 in ihrer Einbaulage im Gehäuse 1, während die untere Zeichnung die Verschiebeplattform 10 als separates Einbaumodul zeigt. An der Unterseite der Verschiebeplattform 10 ist eine Positionier- und Befestigungseinrichtung 16 für den Kraftsensor 13 angeordnet. Diese Einrichtung 16 bildet gleichzeitig die parallelen Stege 16a und 16b aus und eine Führungseinrichtung für den Kraftsensor 13 sowie ein Befestigungsplättchen 15. Der Kraftsensor 13 ist zum Zwecke seiner exakten Positionierung in Bezug auf die Stege 16a und 16b mit zwei seitlichen Einkerbungen 16d versehen, die mit der Führungseinrichtung der Positionier- und Befestigungseinrichtung 16 zusammenwirken. Im Ausführungsbeispiel kommt in die beiden Einkerbungen 16d je ein von der Positionier- und Befestigungseinrichtung 16 abragender Führungsstift zu liegen.

Die Anordnung des Antriebs 7-11 auf der gemeinsamen Verschiebeplattform 10 hat auch fertigungstechnische Vorteile. Hierdurch kann nämlich der Antrieb auf der Verschiebeplattform 10 komplett vormontiert bzw. integriert werden. Anschließend wird die Verschiebeplattform 10 als Ganzes durch eine rückwärtige offene Stirnfläche des Gehäuses 1 in das Gehäuse 1 eingeschoben. Mit dem Einbauen werden auch die erforderlichen Kontakte zur Energieversorgung und Steuerung des Motors 11 hergestellt. Im Ausführungsbeispiel, bei dem auch der Motor 11 zusammen mit der Verschiebeplattform 10 verschiebbar ist, werden die Kontakte durch Steckkontakte an beiden Enden einer Verbindungsleitung gebildet und die Bewegungen von der flexiblen Verbindungsleitung aufgenommen. Es könnten auch die Kontakte für den Motor 11 als Gleitkontakte ausgebildet sein. Nach dem Einsetzen der Verschiebeplattform 10 wird in die rückwärtige, offene Gehäusestirnfläche eine Bodenplatte 1b eingelegt und am Gehäuse 1 fixiert. An der Bodenplatte 1b ist der dritte Steg 16c ausgebildet. Schließlich wird das Gehäuse 1 mittels eines Deckels 1a verschlossen. Die Verschiebeplattform 10 ist nun frei schwimmend zwischen dem dritten Steg 16c einerseits und einer in Verschieberichtung des Kolbens 6 gesehen gegenüberliegenden, gehäuseseitigen Schulterfläche 1c nur durch die O-Ringe 10b dem Gehäuse 1 gegenüber gelagert.

In dieser anfänglichen Einbaulage der Verschiebeplattform 10 besteht noch keine Berührung zwischen der Gewindestange 7 und dem Kolben 6. Solange solch eine Berührung noch nicht stattfindet, d.h. in ihrer anfänglichen Einbaulage, hat die Verschiebeplattform 10 zwischen der gehäuseseitigen Schulterfläche 1c und dem Kraftsensor 13 ein geringes Spiel.

Vom Kraftsensor 13 wird ein die aufgeprägte Reaktionskraft repräsentierender, vorzugsweise zur Reaktionskraft proportionaler Messwert, vorzugsweise in Form eines elektrischen Messwertsignals, über eine Leitung 17 auf eine Steuerung 20 für den Motor 11 ausgegeben. Der die aktuell gemessene Reaktionskraft repräsentierende Messwert liegt permanent an einem Eingang I der Steuerung 20. Über einen Ausgang O und eine entsprechende Steuerungsleitung bzw. einen Steuerungsbus 18 ist die Steuerung 20 mit dem Motor 11 verbunden. Der Motor 11 wird positionsgesteuert.

Der Motor 11 ist ein Schrittmotor mit physikalisch vorgegebener Start-Stop-Frequenz. Hierbei handelt es sich um eine Frequenz und dementsprechende Motordrehzahl, bei deren Überschreiten das Motordrehmoment abnimmt, wodurch der Motor stillgesetzt wird, falls er in diesem Zustand einen vergleichsweise geringen Widerstand erfährt. Nach dem Stillsetzen fährt er nicht mehr selbsttätig an, sondern schwingt nur noch hin und her, bis er ganz abgestellt wird. Durch einen Steuerungsbefehl der Steuerung 20 wird er anschließend wieder angefahren.

Die Position des Motors 11 wird mittels eines auf der Motorrotorachse befestigten Flügelrads 11a und eines damit zusammenwirkenden optischen Sensors, für den das Flügelrad 11a als Lichtschrankenunterbrecher dient, überwacht. Die Steuerung 20 stellt den Motor 11 ab, falls ein Steuerungsimpuls nicht zu einer Motordrehung führt. Der Steuerung 20 ist die Motorposition zumindest in Form der Anzahl der zu jedem Zeitpunkt aus einer Referenzposition heraus zurückgelegten Umdrehungen bekannt. Gegebenenfalls kann die Motorposition auch mittels eines an den optischen Sensor angeschlossenen Zählwerks ermittelt werden, das die Zahl der Unterbrechungen durch die Flügel des Flügelrads hochzählt. Damit kennt die Steuerung auch die Position der Gewindestange 7 relativ zur Verschiebeplattform 10 und letztlich auch die Position des Kolbens 6 in der Ampulle 2.

Die Steuerung 20 umfasst einen Mikroprozessor 21 mit zwei nicht flüchtigen Speichern 22 und 23. Im Speicher 22 ist ein Standard-Sollwertverlauf S für die Reaktionskraft gespeichert. Der weitere Speicher 23 wird bei einem Primen der Infusionspumpe beschrieben. Die Steuerung 20 ist über eine Schnittstelle I/0 mit dem Motor 11, dem Kraftsensor 13 und weiteren Komponenten, insbesondere einer Energiequelle, verbunden. Die Verbindung zum Kraftsensor 13 ist mit dem Bezugszeichen 17 und die zum Motor 11 mit dem Bezugszeichen 18 angedeutet.

Beim Primen wird aus einem Ausgangszustand, in dem die Ampulle 2 in das Gehäuse 1 eingesetzt und ihre Verschlussmembran am Auslass 3 durch eine Verbindungsnadel durchstochen wurde, die Gewindestange 7 auf den Kolben 6 zu verfahren. Bis zum Anstoßen an den Kolben 6 fährt der Motor 11 im Schnellgang deutlich oberhalb der Start-Stop-Frequenz.

Im Schnellgang wird er mit einer Pulsfrequenz betrieben, die vorzugsweise wenigstens doppelt so groß wie seine Start-Stop Frequenz ist. Sobald die Gewindestange 7 mit Ihrem Stempel an den Kolben 6 anstößt, stellt sich der Motor 11 selbsttätig ab, da sein Drehmoment oberhalb der Start-Stop-Frequenz nicht ausreicht, die Gewindestange 7 und den Kolben 6 anzutreiben. In der Steuerung 20 wird die Stopposition des Motors 11 registriert und als Nullposition für die Produktausschüttung gespeichert. Falls für den verwendeten Ampullentyp in der Steuerung 20 ein geeigneter Referenzwert für eine bestimmte Kolbenposition gespeichert ist, kann die Steuerung 20 durch Vergleich mit diesem Referenzwert aus der beim Anfahren an den Kolben festgestellten tatsächlichen Kolbenposition ermitteln, ob es sich bei der Ampulle 2 um eine volle oder beispielsweise halbvolle Ampulle handelt.

Obgleich bevorzugt, muss jedoch kein Schrittmotor mit einstellbarer und dementsprechend voreingestellter Start-Stop-Frequenz verwendet werden. Die Kolbenposition in der Ampulle 2 kann ebenso mittels des Kraftsensors 13 ermittelt werden, da beim Anfahren an den Kolben 6 aufgrund der schwimmenden Lagerung der Verschiebeplattform 10 eine Reaktionskraft sich aufbaut, die letztlich die Verschiebeplattform 10 mit dem Kraftsensor 13 gegen den Steg 16c drückt. Der dabei sich biegende Kraftsensor 30 gibt einen Messwert für die aufgenomme Reaktionskraft aus. Anhand dieses Messwerts kann die Steuerung 20 ebenfalls feststellen, ob die Gewindestange 7 gegen den Kolben 6 gefahren ist und in welcher Position sich zu diesem Zeitpunkt der Kolben 6 in der Ampulle 2 befindet.

Im Ausführungsbeispiel wird der als Schrittmotor mit einstellbarer Start-Stop-Frequenz ausgebildete Motor 11 nach dem vorbeschriebenen Stillsetzen automatisch wieder angefahren, so dass nun der Kolben 6 in der Ampulle 2 vorgeschoben wird. Im Zuge des Primens wird der Kolben 6 in der Ampulle 2 auf den Auslass 3 zu so lange verschoben, bis Insulin an der Austrittsstelle der Infusionsnadel 5 austritt. Sobald der Fluidaustritt sicher festgestellt ist, wird die Gewindestange 7 und damit auch der Kolben 6 in der gerade erreichten Verschiebeposition entlang der gemeinsamen Verschiebeachse V stillgesetzt. Das Stillsetzen kann manuell erfolgen, wenn der Austritt vom Verwender beobachtet wird, oder auch automatisch durch die Steuerung 20.

Durch die erfindungsgemäße Drucküberwachung ist es möglich, das Primen selbst zur Gewinnung eines oder mehrerer Sollwerte oder eines Sollwertverlaufs für die Reaktionskraft zu nutzen, wobei die beim Primen gewonnenen Sollwerte für das jeweilige Injektions- oder Infusionsgerät individuell sind. Um die Individualisierung der Drucküberwachung zu ermöglichen, ist der Speicher 23 beschreibbar, vorzugsweise ist er als sogenannter E²PROM bzw. Flash-ROM ausgebildet. Der Speicher 22 zur Speicherung von Standard-Sollwerten wird durch einen Nur-Lesespeicher gebildet. Grundsätzlich könnten natürlich beide Speicher 22 und 23 durch einen einzigen beschreibbaren Speicher gebildet werden, wobei dem oder den Standard-Sollwert(en) ein eigener Speicherbereich zugewiesen und dieser Speicherbereich von etwaigen Löschvorgängen ausgenommen wäre.

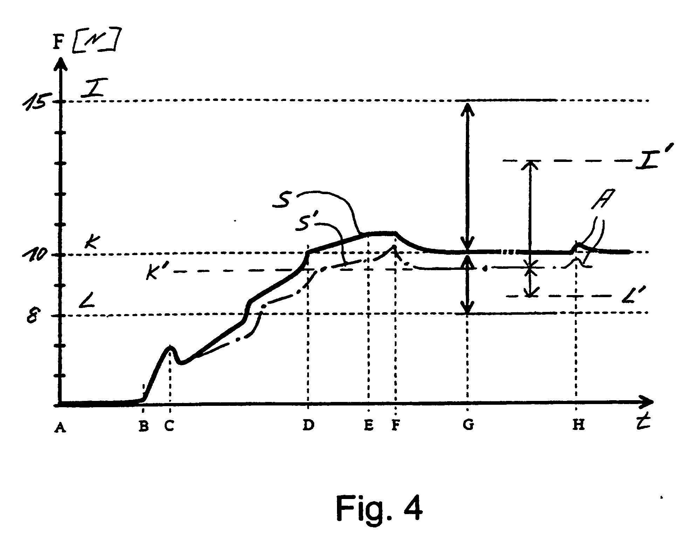

Figur 4 zeigt in einem Zeit-Kraft-Diagramm einen über der Zeit t aufgetragenen Standard-Sollwertverlauf S, wie er im Speicher 22 abgelegt ist. Zusätzlich zu dem in einer durchgezogenen Linie eingetragenen Standard-Sollwertverlauf S ist in strichpunktierter Linie ein Sollwert-Verlauf S' eingetragen, der bei einem Primen sich eingestellt hat und danach noch einstellt. In der Zeit t, gerechnet von einem Zeitpunkt A, der das erste Einschalten des Geräts nach einem Ampullenwechsel markiert, übt der Kolben beim Durchlaufen des eingestellten Programms die Reaktionskraft S' aus. Der Standard-Sollwertverlauf S ist für alle Geräte einer Bauart gleich. Zumindest ein für die Überwachung maßgeblicher Sollwert daraus wird vom Gerätehersteller vorgegeben, d.h. die Infusionspumpe wird derart voreingestellt ausgehändigt. Der Standard-Sollwertverlauf S kann vom Hersteller empirisch oder durch Simulation ermittelt und in den Speicher 22 eingeschrieben werden.

Der Standard Sollwertverlauf S umfasst das Primen und wird auch zu dessen Überwachung herangezogen. Beim Primen wird der für die jeweilige Pumpe individuelle Reaktionskraftverlauf, der danach als individueller Sollwertverlauf S' dient, abgefahren, der sich über die späteren Ausschüttungen A fortsetzt. Der Priming-Vorgang wird aus der Einbaulage, der Referenzposition zum Zeitpunkt A, gestartet. Zum Primen wird die Gewindestange 7 aus der Einbauposition zunächst an den Kolben 6 in die Position zum Zeitpunkt B gefahren. Die Wegstrecke zwischen den Zeitpunkten A nach B durchfährt der Motor 11 im Schnellgang mit beispielsweise 1200 Schritten bzw. Pulsen pro Sekunde (pps=pulses per second). Die Position zum Zeitpunkt B wird festgestellt und gespeichert. Dann fährt der Motor für den gesamten Rest des Programms mit beispielsweise 200 pps weiter. Hat die Reaktionskraft die zur Überwindung der Haftreibung erforderliche Kraft überstiegen, was bei C der Fall ist, setzt sich der Kolben 6 in der Ampulle 2 in Bewegung. Anschließend nimmt die Reaktionskraft weiter zu bis das Ventil 30 mit Produktfluid gefüllt ist, was in der Position zum Zeitpunkt D der Falls ist. Die zur Überwindung des Ventilgegendrucks PV erforderliche Reaktionskraft bzw. deren Sollwert S wird mit K bezeichnet, der beim Primen gemessene Wert mit K'. Danach füllt sich der Katheter stromabwärts von dem Ventil 30. Unmittelbar vor dem ersten Fluidaustritt an der Nadel, wird in der Position zum Zeitpunkt E ein Maximalwert erreicht. Der Motor wird von dem Benutzer bei F gestoppt, nachdem Fluidaustritt beobachtet wurde. Mit erfolgendem Fluidaustritt und insbesondere dem Abschalten des Geräts durch den Benutzer sinkt die Reaktionskraft wieder auf K' ab. Nach dem Feststellen des Fluidaustritts durch Beobachtung oder auch automatisiert durch Feststellen des Absinkens bei G wird die gemessene Kraft K' als individueller Sollwert der Infusionspumpe gespeichert und statt des Standard-Sollwerts K für die spätere Verabreichung verwendet, falls die Relation L < K' < I erfüllt ist. I und L sind die für die Infusionspumpen einer Baureihe zulässigen Grenzwerte der gemessenen Reaktionskraft und werden von dem für die Gerätebaureihe typischen Standard-Sollwert K abgeleitet; L liegt etwa 20% unter und I etwa 50% über K. I ist ein Okklusionsschwellwert, und L ist ein Leckschwellwert. Die dann auftretenden Spitzen repräsentieren die Reaktionskraftwerte für die einzelnen Bolusabgaben zu den Zeitpunkten H der Produktverabreichung.

Grundsätzlich genügt der dem Druckabfall am Ventil entsprechende Standard-Sollwert K mit zulässigen Maximalabweichungen I-K und K-L oder direkt in Form der Bereichsgrenzen I und L als einzige Sollwertvorgabe. Durch die optionale Anpassung dieser Sollwertvorgabe auf den individuellen Sollwert K' kann die Überwachung den tatsächlichen Verhältnissen genauer angepasst und verfeinert werden. Die zulässigen Maximalabweichungen I'-K' und K'-L' zu K' können kleiner sein als die zu K, da gerätebedingte Streuungen durch den Übergang auf den individuellen Sollwert K' bereits kompensiert sind.

Durch einen Vergleich mit dem Standard-Sollwertverlauf S oder auch nur mit dem Sollwert K kann auch das Primen selbst vollständig automatisiert erfolgen. Die erfolgreiche Durchführung des Primens und auch der dabei ermittelte Füllstand der Ampulle 2 können dem Verwender auf einem Display der Infusionspumpe angezeigt werden. Bei Identifizierung etwaiger Okklusionen, vorzugsweise auch von etwaigen Leckagen, wird der Motor 11 von der Steuerung 20 über die Leitung 18 stillgesetzt. Durch entsprechende Anzeigemittel und Alarmmittel können solche Fehlfunktionen dem Verwender zur Kenntnis gebracht werden.

Die erfindungsgemäße Drucküberwachung mittels direkter Kraftmessung und unmittelbarem Vergleich des vom Kraftsensor 13 ausgegebenen Messwerts, der den in der Ampulle 2 herrschenden Fluiddruck repräsentiert, mit seinem Sollwert ermöglicht eine äußerst rasche Identifikation eines unzulässigen Druckzustands. Die Erfindung ermöglicht desweiteren eine erhebliche Verfeinerung der Überwachung dadurch, dass sozusagen auf dem Wege eines Zwischenschritts ein individueller Sollwert oder Sollwert-Verlauf für den Vergleich bei der tatsächlichen Verabreichung gewonnen wird. Hierdurch wird die Zuverlässigkeit der Drucküberwachung erheblich gesteigert. Die erfindungsgemäße Drucküberwachung ist schließlich in mehrfacher Hinsicht nutzbringend verwendbar, indem nicht nur Okklusionen, sondern auch Leckagen identifiziert und darüberhinaus das Primen automatisiert und sogar der Füllzustand der Ampulle automatisch ermittelt werden können.

## Patentansprüche

1. Verfahren zur Ermittlung eines Sollwertes zur Überwachung des Drucks eines bei einer Infusion oder Injektion dosiert zu verabreichenden Produktfluids, welches aus einem Behältnis (2), das in einem Gehäuse (1) aufgenommen ist oder durch dieses selbst gebildet wird, durch Vorschieben eines in dem Behältnis (2) aufgenommen Kolbens (6) ausschüttbar ist, wobei
a) eine als Maß für den Druck dienende, vom Kolben (6) auf das Gehäuse (1) ausgeübte Reaktionskraft gemessen und einer Steuerung (20) für einen Antrieb (7-11) des Kolbens (6) zugeführt wird und
b) die Steuerung (20) die gemessene Reaktionskraft mit einer vorgegebenen Vergleichskraft vergleicht und den Antrieb (7-11) des Kolbens (6) unter Berücksichtigung des Vergleichsergebnisses steuert,
c) die Vergleichskraft ein Sollwert (S; S') für die Reaktionskraft ist und ein unmittelbarer Soll/Ist-Vergleich zwischen der gemessenen Reaktionskraft und ihrem Sollwert (S; S') durchgeführt wird, wobei
d) eine beim Primen des Kolbens gemessene Reaktionskraft mit einem vorgegebenen Standard-Sollwert (S) verglichen wird und bei Einhaltung einer vorgegebenen, maximal zulässigen Differenz zum Standard-Sollwert (S) als neuer Sollwert (S') bei der Verabreichung verwendet wird.

2. Verfahren nach Anspruch 1, **dadurch gekennzeichnet, dass** bei dem Primen der Kolben (6) aus einer Einbauposition im Behältnis (2) bis zu einer Ausschüttungsposition von Produktfluid in eine Ausgangsposition für eine erste Verabreichung vorgeschoben wird, um den Sollwert (S') zu ermitteln.

3. Verfahren nach einem der vorhergehenden Ansprüche, **dadurch gekennzeichnet, dass** in einem Strömungsquerschnitt des Produktfluids zwischen dem Behältnis (2) und einer Infusions- oder Injektionsnadel (5) ein definierter Druckabfall (PC) erzeugt wird.

4. Verfahren nach einem der vorhergehenden Ansprüche, **dadurch gekennzeichnet, dass** bei einem Primen des Kolbens (6), bei dem der Kolben (6) aus einer Einbauposition im Behältnis (2) bis zu einer Ausschüttung von Produktfluid in eine Ausgangsposition für eine erste Verabreichung vorgeschoben wird, ein erstes Anstoßen des Antriebs (7-11) an dem Kolben (6) festgestellt und aus der Position des Antriebs (7-11) bei dem Anstoßen der Füllzustand des Behältnisses (2) ermittelt wird.

5. Verfahren nach einem der vorhergehenden Ansprüche, **dadurch gekennzeichnet, dass** eine Start-Stop-Frequenz eines Schrittmotors (11) des Antriebs (7-11) so eingestellt ist, dass der Schrittmotor (11) bei einem ersten Anstoßen des Antriebs (7-11) an den Kolben (6) festgesetzt wird, und dass aus einer bei dem Anstoßen eingenommenen Position des Motors (11) ein Füllzustand des Behältnisses (2) ermittelt wird.

6. Verfahren nach einem der vorhergehenden Ansprüche, **dadurch gekennzeichnet, dass** wenigstens ein erster Sollwert und ein zweiter Sollwert für die Reaktionskraft vorgegeben werden, wobei der erste Sollwert eine vom Kolben (6) für ein Verschieben aus einer Ruheposition auszuübende Reaktionskraft und der zweite Sollwert eine sich bei der Verschiebebewegung des Kolbens (6) stationär einstellende Reaktionskraft repräsentieren.

7. Vorrichtung zur Überwachung des Drucks eines Produktfluids basierend auf einer Ermittlung eines Sollwertes, wobei das Produktfluid zum Zwecke einer Infusion oder Injektion aus einem Behältnis (2) durch Vorschieben eines im Behältnis (2) aufgenommenen Kolbens (6) dosiert ausschüttbar ist, wobei die Vorrichtung umfasst:
a) ein das Behältnis (2) aufnehmendes oder dieses selbst bildendes Gehäuse (1),
b) einen Kraftsensor (13), der eine als Maß für den Druck dienende, vom Kolben (6) auf das Gehäuse (1) ausgeübte Reaktionskraft misst und als Messwert ausgibt, und
c) eine Steuerung (20), die den Messwert mit einem vorgegebenen Vergleichswert vergleicht und einen Antrieb (7-11) für den Kolben (6) unter Berücksichtigung des Vergleichsergebnisses steuert,
d) wobei wenigstens ein Sollwert (S; S') für die Reaktionskraft in einem Speicher (22, 23) der Steuerung (20) gespeichert ist und für einen unmittelbaren Soll/Ist-Vergleich mit der gemessene Reaktionskraft verwendet wird, und
e) einen ersten Speicher (22), in dem ein Standard-Sollwert (S) für die Reaktionskraft gespeichert ist, wobei die Steuerung (20) geeignet ist, die bei einem Primen des Kolbens (6) Kraftsensor (13) gemessene Reaktionskraft mit dem Standard-Sollwert (S) zu vergleichen und bei Einhaltung einer vorgegebenen, maximal zulässigen Differenz zwischen dem Standard-Sollwert (S) und dieser Reaktionskraft diese in einem zweiten Speicher (23) der Steuerung (20) zu speichern, wobei der zweite Speicher (23) geeignet ist, die Reaktionskraft zu speichern, und diese als für die Vorrichtung individuellen Sollwert (S') für den Soll/Ist-Vergleich bei der Infusion oder Injektion zu verwenden.

8. Vorrichtung nach Anspruch 7, **dadurch gekennzeichnet, dass** der Kraftsensor (13) in der Flucht einer Verschiebeachse (V) des Kolbens (6) angeordnet ist.

9. Vorrichtung nach einem der Ansprüche 7 oder 8, **dadurch gekennzeichnet, dass** ein auf den Kolben (6) unmittelbar wirkendes Abtriebsglied (7) und ein mit dem Abtriebsglied (7) gekoppeltes Antriebsglied des Antriebs (7-11) schwimmend geradverschiebbar im Gehäuse (1) gelagert sind.

10. Vorrichtung nach dem vorangehenden Anspruch, **dadurch gekennzeichnet, dass** ein Motor (11) zum Antreiben des Abtriebsglieds schwimmend geradverschiebbar ist.

11. Vorrichtung nach einem der Ansprüche 7 bis 10, **dadurch gekennzeichnet, dass** der Kraftsensor (13) einen Biegebalken (14) umfasst, dessen Biegung in Folge der auf ihn ausgeübten Reaktionskraft auf einen Dehnungssensor (15) wirkt, der den seiner Dehnung oder Stauchung entsprechenden Messwert ausgibt.

12. Vorrichtung nach einem der Ansprüche 7 bis 11, **dadurch gekennzeichnet, dass** ein Ventil (30) in einem Strömungsquerschnitt des Produktfluids zwischen dem Behältnis (2) und einer Infusions- oder Injektionsnadel (5) angeordnet ist, das einen Durchfluss des Produktfluids in Richtung auf die Nadel (5) nur bei Überschreiben eines durch das Ventil (30) definierten Überdrucks zulässt.

## Claims

1. A process for determining a nominal value for monitoring the pressure of a product fluid to be administered in dosed amounts in an infusion or injection, wherein said product fluid can be dispensed from a container (2), received in a housing (1) or formed by the housmg (1) itself, by advancing a piston (6) received in the container (2), wherein:
a) a reaction force exerted by the piston (6) on the housing (1) and serving as a measure for the pressure is measured and fed to a control (20) for a drive (7-11) of the piston (6); and
b) the control (20) compares the measured reaction force with a predetermined reference force and controls the drive (7-11) of the piston (6) in consideration of the result of the comparison;
c) the reference force is a nominal value (S; S') for the reaction force and a direct nominal/actual comparison is carried out between the measured reaction force and its nominal value (S; S'), wherein
d) a reaction force measured when priming the piston is compared with a predetermined standard nominal value (S) and, if within a predetermined, maximum permissible difference to the standard nominal value (S), is used as a new nominal value (S') when administering.

2. The process according to claim 1, **characterised in that** during priming, the piston (6) is advanced from an installation position in the container (2), up to an expulsion of product fluid position, to a starting position for a first administration, in order to determine the nominal value (S').

3. The process according to any one of the preceding claims, **characterised in that** a defined pressure drop (PC) is produced in a flow cross section of the product fluid between the container (2) and an infusion or injection needle (5).

4. The process according to any one of the preceding claims, **characterised in that** during a priming of the piston (6), in which the piston (6) is advanced from an installation position in the container (2), up to an expulsion of product fluid, to a starting position for a first administration, a first striking of the drive (7-11) against the piston (6) is detected and the fill level of the container (2) is determined from the position of the drive (7-11) upon contact.

5. The process according to any one of the preceding claims, **characterised in that** a start-stop frequency of a step motor (11) of the drive (7-11) is adjusted such that the step motor (11) is arrested at a first contact of the drive (7-11) against the piston (6), and m that a fill level of the container (2) is determined from a position of the motor (11) assumed upon contact.

6. The process according to any one of the preceding claims, **characterised in that** at least a first nominal value and a second nominal value for the reaction force are predetermined, wherein the first nominal value represents a reaction force to be exerted by the piston (6) for a displacement from a resting position, and the second nominal value represents a reaction force which adopts a constant value during the displacement movement of the piston (6).

7. An apparatus for monitoring the pressure of a product fluid, based on determining a nominal value, wherein the product fluid can be dispensed in dosed amounts from a container (2), for the purpose of an infusion or injection, by advancing a piston (6) received in the container (2), wherein the apparatus comprises:
a) a housing (1) which receives the container (2) or itself forms the container (2);
b) a force sensor (13) which measures a reaction force serving as a measure of the pressure and exerted by the piston (6) on the housing (1) and outputs it as a measured value; and
c) a control (20) which compares the measured value with a predetermined reference value and controls a drive (7-11) for the piston (6) in consideration of the result of the comparison;
d) wherein at least one nominal value (S; S') for the reaction force is stored in a memory (22, 23) of the control (20) and used for a direct nominal/actual comparison with the measured reaction force.

8. The apparatus according to claim 7, **characterised in that** the force sensor (13) is positioned in alignment with an axis of displacement (V) of the piston (6).

9. The apparatus according to any one of claims 7 or 8, **characterised in that** a driven member (7) acting directly on the piston (6) and a drive member of the drive (7-11) coupled directly to the driven member (7) are supported floating and such that they can be linearly displaced in the housing (1).

10. The apparatus according to the preceding claim, **characterised in that** a motor (11) is floating and can be linearly displaced in order to drive the driven member.

11. The apparatus according to any one of claims 7 to 10, **characterised in that** the force sensor (13) includes a bendable beam (14) which, when bent as a result of the reaction force exerted on it, acts on a strain gauge (15) which outputs the measured value corresponding to its stretching or compression.

12. The apparatus according to any one of claims 7 to 11, **characterised in that** a valve (30) is positioned in a flow cross section of the product fluid between the container (2) and an infusion or injection needle (5) and only permits the product fluid to flow towards the needle (5) when a pressure burden defined by the valve (30) is exceeded.

## Revendications

1. Procédé pour déterminer une valeur de consigne pour surveiller la pression d'un produit fluide administré par dose lors d'une perfusion ou d'une injection, produit qui, depuis un récipient (2), qui est reçu dans un boîtier (1) ou qui est formé lui-même par celui-ci, peut être distribué par avance d'un piston (6) reçu dans le récipient (2), dans lequel :
a) on mesure, à titre de grandeur pour la pression, une force de réaction qui est exercée par le piston (6) sur le boîtier (1), et on la fournit à une commande (20) pour un entraînement (7-11) du piston (6),
b) la commande (20) compare la force de réaction mesurée à une force comparative prédéterminée et commande l'entraînement (7-11) du piston (6) en tenant compte du résultat de comparaison,
c) la force comparative est une valeur de consigne (S ; S') pour la force de réaction et l'on exécute une comparaison directe valeur de consigne/valeur réelle entre la force de réaction mesurée et sa valeur de consigne (S ; S'), et
d) une force de réaction mesurée lors de l'amorçage du piston est comparée à une valeur de consigne standard prédéterminée (S) et, en cas de maintien d'une différence maximale admissible prédéterminée par rapport à la valeur de consigne standard (S), celle-ci est utilisée comme une nouvelle valeur de consigne (S') lors de l'administration.

2. Procédé selon la revendication 1, **caractérisé en ce que** lors de l'amorçage, le piston (6) est avancé depuis une position de montage dans le récipient (2) jusqu'à une position de distribution du produit fluide dans une position de départ pour une première administration, afin de déterminer la valeur de consigne (S').

3. Procédé selon l'une des revendications précédentes, **caractérisé en ce que** l'on engendre une chute de pression définie (PC) dans une section d'écoulement du produit fluide entre le récipient (2) et une aiguille de perfusion ou d'injection (5).

4. Procédé selon l'une des revendications précédentes, **caractérisé en ce que** lors d'un amorçage du piston (6), lors duquel le piston (6) est avancé depuis une position de montage dans le récipient (2) jusqu'à une distribution du produit fluide dans une position de départ pour une première administration, on constate une première venue en butée de l'entraînement (7-11) contre le piston (6), et l'on détermine l'état de remplissage du récipient (2) à partir de la position de l'entraînement (7-11) lors de la venue en butée.

5. Procédé selon l'une des revendications précédentes, **caractérisé en ce que** l'on établit une fréquence démarrage/arrêt d'un moteur pas à pas (11) de l'entraînement (7-11) de telle façon que le moteur pas à pas est immobilisé lors d'une première venue en butée (7-11) contre le piston (6), et **en ce que** l'on détermine un état de remplissage du récipient (2) à partir d'une position du moteur (11) occupée lors de la venue en butée.

6. Procédé selon l'une des revendications précédentes, **caractérisé en ce que** l'on impose au moins une première valeur de consigne et une seconde valeur de consigne pour la force de réaction, la première valeur de consigne représentant une force de réaction à exercer par le piston (6) pour un déplacement à partir d'une position de repos, et la seconde valeur de consigne représentant une force de réaction qui s'établit de façon stationnaire lors du mouvement de déplacement du piston (6).

7. Dispositif pour surveiller la pression d'un produit fluide en se basant sur une détermination d'une valeur de consigne, le produit fluide pouvant être distribué de façon dosée, dans le but d'une perfusion ou d'une injection, hors d'un récipient (2) par avance d'un piston (6) reçu dans le récipient (2), le dispositif comprenant :
a) un boîtier (1) qui reçoit le récipient (2) ou qui forme lui-même celui-ci,
b) un détecteur de force (13) qui mesure, à titre de grandeur pour la pression, une force de réaction exercée par le piston (6) sur le boîtier (1) et qui la fournit à titre de valeur mesurée, et
c) une commande (20) qui compare la valeur mesurée à une valeur comparative prédéterminée et qui commande un entraînement (7-11) pour le piston (6) en tenant compte du résultat de comparaison,
d) au moins une valeur de consigne (S ; S') pour la force de réaction est mémorisée dans une mémoire (22, 23) de la commande (20) et est utilisée pour une comparaison directe valeur de consigne/valeur réelle avec la force de réaction mesurée, et
e) une première mémoire (22), dans laquelle est mémorisée une valeur de consigne standard (S) pour la force de réaction, la commande étant adaptée à comparer la force de réaction mesurée par un capteur de force (13) lors de l'amorçage du piston (6) à la valeur de consigne standard (3), et lors du maintien d'une différence maximale admissible prédéterminée entre la valeur de consigne standard (S) et cette force de réaction, à mémoriser celle-ci dans une seconde mémoire (23) de la commande (20), ladite seconde mémoire (23) étant adaptée à mémoriser la force de réaction et à utiliser celle-ci comme valeur de consigne individuelle (S') pour le dispositif pour la comparaison valeur de consigne/valeur réelle lors de la perfusion ou de l'injection.

8. Dispositif selon la revendication 7, **caractérisé en ce que** le capteur de force (13) est agencé dans l'alignement d'un axe de translation (V) du piston (6).

9. Dispositif selon l'une ou l'autre des revendications 7 et 8, **caractérisé en ce qu'**un organe d'entraînement (7) agissant directement sur le piston (6), et un organe d'entraînement de l'entraînement (7-11) couplé audit organe entraînement (7), sont montés de façon flottante et en translation rectiligne dans le boîtier (1).

10. Dispositif selon la revendication précédente, **caractérisé en ce qu'**un moteur (11) pour l'entraînement de l'organe entraînement est monté de façon flottante et en translation rectiligne.

11. Dispositif selon l'une des revendications 7 à 10, **caractérisé en ce que** le capteur de force (13) comprend une barre de flexion (14), dont la flexion résultant de la force de réaction exercée sur elle-même agit sur une jauge de contraintes (15) qui délivre une valeur mesurée correspondant à son allongement ou à son rétrécissement.

12. Dispositif selon l'une des revendications 7 à 11, **caractérisé en ce qu'**une valve (30) est agencée dans une section d'écoulement du produit fluide entre le récipient (2) et une aiguille de perfusion ou d'injection (5), valve qui permet un écoulement du produit fluide en direction de l'aiguille (5) uniquement lors du dépassement d'une surpression définie par la valve (30).
